# EUROPEAN PATENT APPLICATION

(11) **EP 4 324 516 A1**
(43) Date of publication of application: **21.02.2024**
(21) Application number: 22871950.6
(22) Date of filing: 20.09.2022
(51) Int. Cl.: A61N 5/10

(54) **NEUTRON CAPTURE THERAPY SYSTEM**

(30) Priority: 26.09.2021 CN 202122331728 U; 26.09.2021 CN 202111128771
(71) Applicant: Neuboron Therapy System Ltd., Xiamen, Fujian 361026 (CN)
(72) Inventor: LIU, Yuan-Hao, Nanjing, Jiangsu 211112 (CN); LIN, Chun-Ting, Nanjing, Jiangsu 211112 (CN)
(74) Representative: Gunzelmann, Rainer
(86) International application number: PCT/CN2022/119798
(87) International publication number: WO 2023/045904

(57) **Abstract**

A neutron capture therapy system (100), comprising a charged particle beam generation portion (11), a beam transmission portion (12), and a neutron beam generation portion (13). The neutron beam generation portion (13) comprises a target material (T); the target material (T) interacts with a charged particle beam (P) generated by the charged particle beam generation portion (11) to generate a neutron beam (N); the neutron capture therapy system (100) further comprises a charged particle beam generation chamber (102) and a target material replacement chamber (40); the charged particle beam generation chamber (102) at least partially accommodates the charged particle beam generation portion (11) and the beam transmission portion (12); the target material replacement chamber (40) is used for mounting the target material (T) and allowing the beam transmission portion (12) to pass through the target material replacement chamber (40); the target material replacement chamber (40) is formed behind the charged particle beam generation portion (11) on a beam transmission path and is different from the charged particle beam generation chamber (102). By providing the target material replacement chamber (40) to mount the target material (T), the environment in the target material replacement chamber (40) can be set, so that the oxidation or nitridation of the target material (T) during a mounting process is avoided, the operation is simple, and the cost is low.

## Description

### TECHNICAL FIELD

The present disclosure relates to a radiation irradiation system, in particular a neutron capture therapy system.

### BACKGROUND

With the development of atomic science, radiation therapy, such as cobalt-60 radiation therapy, linear accelerator therapy and electron beam radiation therapy, has become one of the main means of cancer therapy. However, the traditional photon or electron therapy is limited by physical conditions of the radiation itself, which also damages a large amount of normal tissues in the beam path while killing tumor cells. In addition, due to different sensitivity of tumor cells to radiation, the traditional radiation therapy is often ineffective for therapy of malignant tumors with relatively high radiation resistance (e. g., glioblastoma multiforma and melanoma).

In order to reduce radiation damage to normal tissues surrounding tumors, the concept of target therapy in chemotherapy is applied to radiation therapy; for tumor cells with high radiation resistance, radiation sources with high relative biological effectiveness (RBE), such as proton therapy, heavy particle therapy and neutron capture therapy, are also actively developed at present. The neutron capture therapy combines the above two concepts, such as boron neutron capture therapy, which provides a better selection of cancer therapy than conventional radiation therapy by means of specific aggregation of a boron-containing drug in tumor cells, and in conjunction with precise neutron beam regulation.

In the neutron capture therapy system with an accelerator, a charged particle beam is accelerated by the accelerator to have an energy sufficient to overcome a Coulomb repulsive force of a target atom nucleus, and then reacts with the target to generate neutrons. In the process of generating neutrons, the target is irradiated by the high-power accelerated charged particle beam, resulting in the temperature of the target is greatly increased, thereby affecting the service life of the target. In order to stably generate neutrons, old targets need to be replaced with new targets, and Li, which is often used as an action layer of the target, is extremely easily oxidized or nitrided in air, resulting in reduction of the effect of the targets, while an oxidation resistant layer arranged outside the action layer adds cost and consumes some of the energy of the charged particle beam. Therefore, how to install a new target without being oxidized is very important, and a glove box is generally used to perform Li operation in a space filled with an inert gas, however, it is difficult and costly to install the target.

### SUMMARY

In order to solve the foregoing technical problems, one aspect of the present disclosure provides a neutron capture therapy system, including a charged particle beam generation portion, a beam transmitting portion, and a neutron beam generation portion, where the charged particle beam generation portion is configured for generating a charged particle beam, the beam transmission portion transmits the charged particle beam to the neutron beam generation portion, the neutron beam generation portion includes a target that interacts with the charged particle beam to generate a neutron beam, the neutron capture therapy system further includes a charged particle beam generation chamber and a target replacement chamber, where the charged particle beam generation chamber at least partly houses the charged particle beam generation portion and the beam transmission portion, the target replacement chamber is configured for installing the target and passing the beam transmission portion through the target replacement chamber, and the target replacement chamber is formed behind the charged particle beam generation portion in a transmission path of the charged particle beam and is different from the charged particle beam generation chamber. By providing the target replacement chamber for installing the target, the environment in the target replacement chamber can be set, so that the target is protected from being oxidized or nitrided during the installing process, and the operation is simple and the cost is low.

Preferably, the target includes an action layer, the action layer interacts with the charged particle beam to generate the neutron beam, and the action layer is pre-housed in the target replacement chamber.

Furthermore, a material of the action layer is Li or an alloy thereof or a compound thereof, the charged particle beam is a proton beam, and the proton beam is performed a nuclear reaction of ⁷Li(p, n)⁷Be with the action layer to generate the neutron beam.

Preferably, an environment in the target replacement chamber has a dew point temperature of Td less than or equal to -30°C at atmospheric pressure or a relative humidity RH less than or equal to 0.6% at 25 °C.

Preferably, the target includes a cooling passage, and the neutron capture therapy system further includes a first cooling tube and a second cooling tube both connected to the cooling passage.

Preferably, the neutron beam generation portion further includes a beam shaping portion capable of adjusting a quality of the neutron beam generated by an interaction of the charged particle beam and the target; the neutron capture therapy system further includes an irradiation chamber in which a subject to be irradiated is performed therapy by irradiation of the neutron beam, the beam shaping portion is at least partially housed in a separation wall configured for separating the irradiation chamber and the charged particle beam generation chamber, the target is installed in the beam shaping portion, and the target replacement chamber is arranged in the charged particle beam generation chamber.

Furthermore, the charged particle beam generation chamber has a ceiling plate and a floor plate, the target replacement chamber is a semi-sealed dehumidification area enclosed by the separation wall, the ceiling plate, the floor plate, and a curtain arranged between the ceiling plate and the floor plate, and a dehumidifier is arranged in the semi-sealed dehumidification area; alternatively, the target replacement chamber includes a top, the target replacement chamber is a semi-sealed dehumidification area enclosed by the separation wall, the top, the floor plate and a curtain arranged between the top and the floor plate, and a dehumidifier is arranged in the semi-sealed dehumidification area.

Furthermore, the curtain includes multiple curtain pieces sequentially arranged without gaps; the curtain is made of a material which, when irradiated by neutrons, generates a product having no radioactivity or low radioactivity, or a radioisotope with a short half-life; the dehumidifier includes a shell which is made of a neutron shielding material or is provided with a radiation shield outside the dehumidifier.

Preferably, the beam transmission portion includes a transmission tube having an adjustable length, the target is arranged on an end of the transmission tube, and the end of the transmission tube on which the target is arranged is located in the target replacement chamber.

Furthermore, the beam transmission portion includes a first transmission tube, a second transmission tube and a third transmission tube, where the second transmission tube is detachably connected to the first transmission tube and the third transmission tube, and the target is provided on an end of the first transmission tube, the first transmission tube and at least part of the second transmission tube are arranged in the target replacement chamber, the second transmission tube or the third transmission tube passes through the target replacement chamber.

Furthermore, a beam adjusting device is disposed on the third transmission tube, and a detection device is disposed on the first transmission tube.

A second aspect of the present disclosure provides an installing method for a target of a particle beam generation device, the target includes an action layer configured for generating a particle beam, the particle beam generation device includes a transmission tube, the target is installed on an end of the transmission tube, the installing method includes: pre-housing the action layer in a target replacement chamber; installing the target on the end of the transmission tube in the target replacement chamber; installing the target to the particle beam generation device in the target replacement chamber. By providing the target replacement chamber for installing the target, the environment in the target replacement chamber can be set, so that the target is protected from being oxidized or nitrided during the installing process, and the operation is simple and the cost is low

Preferably, the transmission tube includes a first transmission tube and a second transmission tube, the target is installed on an end of the first transmission tube, and a length of the second transmission tube is adjustable, the target is installed to the particle beam generation device by connecting the first transmission tube to the second transmission tube and adjusting the length of the second transmission tube in the target replacement chamber.

Preferably, the particle beam generation device further includes a beam shaping portion, the target is arranged in the beam shaping portion, the target includes a cooling passage, the particle beam generation device further includes a first cooling tube and a second cooling tube both connected to the cooling passage, the installing method further includes: connecting the first cooling tube and the second cooling tube to the cooling passage of the target in the target replacement chamber; inserting the end of the transmission tube, on which the target is installed, into the beam shaping portion in the target replacement chamber; connecting the first cooling tube and the second cooling tube to an external cooling source.

Preferably, an environment in the target replacement chamber has a dew point temperature of Td less than or equal to -30°C at atmospheric pressure or a relative humidity RH less than or equal to 0.6% at 25 °C.

The neutron capture therapy system and the installing method for a target of a particle beam generation device of the present disclosure are provided. By providing the target replacement chamber for installing the target, so that the target is protected from being oxidized or nitrided during the installing process, and the operation is simple and the cost is low.

### BRIEF DESCRIPTION OF THE DRAWINGS

Fig. 1 is a schematic structural view of a neutron capture therapy system according to an embodiment of the present disclosure;
Fig. 2 is a schematic structural view of a target according to an embodiment of the present disclosure;
Fig. 3 is a schematic view of a charged particle beam generation chamber and a target replacement chamber in Fig. 1 viewed in another direction;
Fig. 4 is a schematic view of a charged particle beam generation chamber and a target replacement chamber according to another embodiment of the present disclosure; and
Fig. 5 is a flowchart of a method for installing a target according to an embodiment of the present disclosure.

### DETAILED DESCRIPTION OF THE EMBODIMENTS

The present disclosure is further described in detail below with reference to the accompanying drawings, so that those skilled in the art can implement the present disclosure according to the text of the specification.

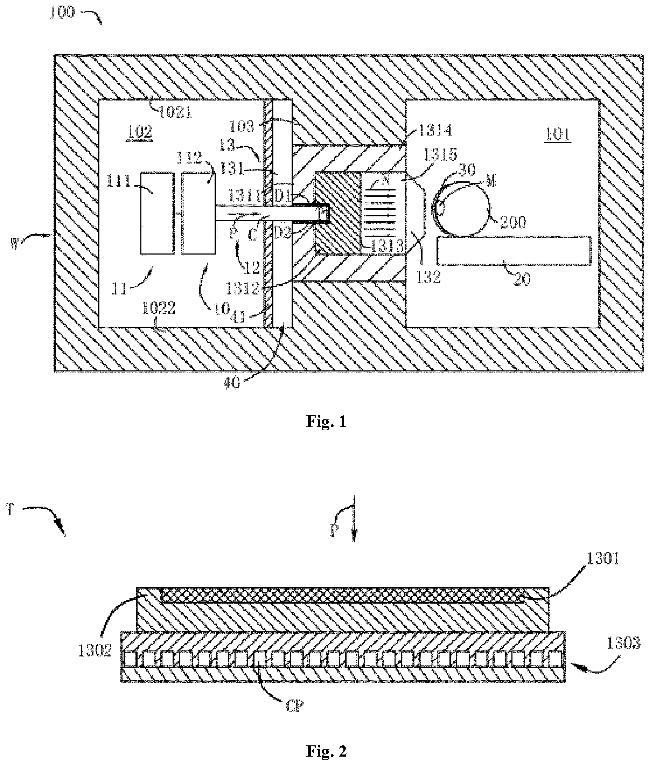

As shown in Fig. 1, a neutron capture therapy system in this embodiment is preferably a boron neutron capture therapy system 100. The boron neutron capture therapy system 100 is a device for performing cancer therapy by means of a boron neutron capture therapy. The boron neutron capture therapy carries out the cancer therapy by irradiating a neutron beam N to a subject to be irradiated 200 injected with boron (B-10). Upon taking or injecting the boron-containing (B-10) drug into the subject to be irradiated 200, the boron-containing drug selectively accumulates in tumor cells M. Based on a high capture cross section property for thermal neutrons of the boron-containing (B-10) drug, two heavy charged particles of ⁴He and ⁷Li are generated by a neutron capture and nuclear fission reaction of ¹⁰B(n, α)⁷Li. The average energy of the two charged particles of ⁴He and ⁷Li is about 2.33MeV, and the two charged particles have high Linear Energy Transfer (LET) and short distance properties, wherein the Linear Energy Transfer and distance of the alpha short particle are 150keV/µm and 8µm, respectively, while that of the heavy particle of ⁷Li are 175keV/µm and 5µm, respectively, the total distance of the two heavy particles is approximately equivalent to a cell size, and therefore the radiation damage to organisms is limited to the cell level, which can achieve the purpose of locally killing tumor cells without causing too much damage to normal tissues.

The boron neutron capture therapy system 100 includes a neutron beam generation device 10 and a treatment station 20. The neutron beam generation device 10 includes a charged particle beam generation portion 11, a beam transmission portion 12, and a neutron beam generation portion 13. The charged particle beam generation portion 11 generates a charged particle beam P such as a proton beam; the beam transmission portion 12 transmits the charged particle beam P to the neutron beam generation portion 13; the neutron beam generation portion 13 generates a neutron beam N for therapy and irradiates the subject to be irradiated 200 on the treatment station 20.

The charged particle beam generation portion 11 can include an ion source 111 configured for generating charged particles such as H⁻, proton, deuteron, and an accelerator 112 configured for accelerating the charged particles generated by the ion source 111 to obtain the charged particle beam P of a desired energy, such as a proton beam.

The neutron beam generation portion 13 can include a target T, a beam shaping portion 131, and a collimator 132. The charged particle beam P generated by the accelerator 112 irradiates the target T through the beam transmission portion 12 and interacts with the target T to generate neutrons. The generated neutrons form the neutron beam N for therapy through the beam shaping portion 131 and the collimator 132 in sequence, and irradiate the subject to be irradiated 200 on the treatment station 20. The target T is preferably a metal target. A suitable nuclear reaction is selected according to the required neutron yield and energy, energy and current of the available accelerated charged particles, and physical and chemical properties of the metal target, and the nuclear reactions commonly discussed are ⁷Li(p, n)⁷Be and ⁹Be(p, n)⁹B, which are both endothermic reactions. The energy threshold values of the two nuclear reactions are 1.881MeV and 2.055MeV, respectively, and since the ideal neutron source for the boron neutron capture therapy is epithermal neutrons of keV energy level, and theoretically, if the Li target is bombarded with protons whose energy is only slightly higher than the energy threshold value, it is possible to generate neutrons of relatively low energy, which can be used in the clinic without too much slow speed treatment, however, the proton action cross sections of the two targets of lithium metal (Li) and beryllium metal (Be) with the threshold energy are not high, therefore protons of higher energy are usually chosen to initiate the nuclear reaction in order to generate a sufficiently large flux of neutrons. An ideal target should have properties such as a high neutron yield, a distribution of energy of the generated neutron close to the energy region of the epithermal neutron (which will be described in detail below), no generation of too many long-distance radiation, low cost, safety, easy operation and high temperature resistance, but actually a nuclear reaction meeting all requirements cannot be found. As well known to a person skilled in the art, the target T can also be made of a metal material other than Li and Be, such as Ta or W and an alloy thereof, a compound thereof. The accelerator 10 can be a linear accelerator, a cyclotron, a synchrotron, a synchrocyclotron.

The beam shaping portion 131 can adjust the quality of the neutron beam N generated by the interaction of the charged particle beam P and the target T. The collimator 132 is configured for converging the neutron beam N, so that the neutron beam N has a higher targeting ability in the therapy process. The beam shaping portion 131 further includes a reflecting portion 1311, a retarding portion 1312, a thermal neutron absorbing portion 1313, a radiation shielding portion 1314, and a beam outlet 1315. Neutrons generated by the interaction of the charged particle beam P and the target T have a wide energy spectrum, wherein in addition to the epithermal neutron meeting the therapy requirement, the proportion of other types of neutrons and photon needs to be minimized to avoid damage to the operator or the subject to be irradiated, therefore, the neutrons bombarded out from the target T need to pass through the retarding portion 1312 to adjust the fast neutron energy (more than 40keV) therein to the epithermal neutron energy region (0.5 eV - 40keV) and minimize thermal neutrons (less than 0.5eV), the retarding portion 1312 is made of a material having a larger action cross section with the fast neutron and a smaller action cross section with epithermal neutron, the retarding portion 1312 is made of at least one of D₂O, AlF₃, Fluental, CaF₂, Li₂CO₃, MgF₂ and Al₂O₃. The reflecting portion 1311 surrounds the retarding portion 1312, reflects neutrons passing through the retarding portion 1312 and diffusing around back into the neutron beam N so as to improve the utilization rate of neutrons, and is made of a material having strong neutron-reflecting capacity. In the embodiment, the reflecting portion 1311 is made of at least one of Pb or Ni. The thermal neutron absorbing portion 1313 is arranged behind the retarding portion 1312, and is made of a material having a large action cross section with the thermal neutron. In the embodiment, the thermal neutron absorbing portion 1313 is made of Li-6, the thermal neutron absorbing portion 1313 is configured for absorbing the thermal neutrons passing through the retarding portion 1312, so as to reduce the proportion of the thermal neutrons in the neutron beam N, thus avoiding excessive doses from being applied to superficial normal tissues during the therapy. Alternatively, the thermal neutron absorbing portion can be integrated with the retarding portion, the material of the retarding portion contains Li-6. The radiation shielding portion 1314 is configured for shielding neutrons and photons leaked from parts other than the beam outlet 1315. The material of the radiation shielding portion 1314 includes at least one of a photon shielding material and a neutron shielding material. In the embodiment, the material of the radiation shielding portion 1314 includes a photon shielding material plumbum (Pb) and a neutron shielding material polyethylene (PE). Alternatively, the beam shaping portion 131 can have other configurations, as long as the epithermal neutron beam required for therapy can be obtained. The collimator 132 is arranged behind the beam outlet 1315, the epithermal neutron beam coming out from the collimator 132 irradiates the subject to be irradiated 200, pass through the superficial normal tissues, and then is retarded thermal neutrons to reach the tumor cells M. Alternatively, the collimator 132 can be cancelled or replaced by other structures, in this case, the neutron beam comes out from the beam outlet 1315 and directly irradiates the subject to be irradiated 200. In the embodiment, a radiation shielding device 30 is further arranged between the subject to be irradiated 200 and the beam outlet 1315, for shielding the radiation of the beam coming out from the beam outlet 1315 to the normal tissues of the subject to be irradiated. Alternatively, the radiation shielding device 30 cannot be provided. The target T is arranged between the beam transmission portion 12 and the beam shaping portion 131, and the beam transmission portion 12 has a transmission tube C for accelerating or transmitting the charged particle beam P. In the embodiment, the transmission tube C extends into the beam shaping portion 131 in the direction of the charged particle beam P, and sequentially passes through the reflecting portion 1311 and the retarding portion 1312, the target T is arranged in the retarding portion 1312 and located at the end of the transmission tube C, so as to obtain a neutron beam with a better quality. Alternatively, other arrangements of the target are possible.

The boron neutron capture therapy system 100 is housed entirely within a concrete-constructed building. Specifically, the boron neutron capture therapy system 100 further includes an irradiation chamber 101 and a charged particle beam generation chamber 102. The subject to be irradiated 200 on the treatment station 20 is performed therapy by means of irradiation of the neutron beam N in the irradiation chamber 101, the charged particle beam generation chamber 102 at least partly houses the charged particle beam generation portion 11 and the beam transmission portion 12, and the beam shaping portion 20 is at least partially housed in the separation wall 103 separating the irradiation chamber 101 and the charged particle beam generation chamber 102.

The structure of the target T will be described in detail below in conjunction with Fig. 2.

The target T includes an action layer 1301, an anti-foaming layer 1302, and a heat dissipation layer 1303. The action layer 1301 interacts with the charged particle beam P to generate the neutron beam. During the process of generating neutrons, the target is subject to irradiation of the accelerated charged particle beam P with a very high energy level, so that the target is foamed and the temperature thereof is increased, thereby reducing the service life of the target. The anti-foaming layer 1302 is arranged behind the action layer 1301 in the incidence direction of the charged particle beam P and can rapidly diffuse hydrogen generated by the interaction of the charged particle beam P on the target T, so as to reduce the concentration of hydrogen or release hydrogen to the outside, thereby effectively inhibiting foaming caused by the charged particle beam P, thus avoiding or reducing deformation of the target T caused by the foaming, and prolonging the service life of the target. The anti-foaming layer 1302 is made of a material inhibiting foaming, such as a material having a hydrogen diffusion coefficient of not less than 10⁻⁶cm ²/s at 200°C. In an embodiment, the material of the anti-foaming layer 1302 includes at least one of Nb, Ta, Pd, V, alloys and compounds thereof. The heat dissipation layer 1303 is arranged behind the anti-foaming layer 1303 in the incident direction of the charged particle beam P and transfers the heat deposited in the target to be discharged via the cooling medium, thereby reducing the temperature of the target, preventing the target from being deformed due to the excessively high temperature, and prolonging the service life of the target. The heat dissipation layer 1303 is made of a thermally conductive material. In an embodiment, the material of the heat dissipation layer 1303 includes at least one of Cu, Fe, Al, alloys and compounds thereof. Alternatively, the anti-foaming layer 1302 can be made of a material that can not only conduct heat but also inhibit foaming, and in this case, the anti-foaming layer 1302 and the heat dissipation layer 1303 can be integrated. A heat conduction layer can also be provided to directly and quickly transfer the heat deposited in the action layer 1301 to the heat dissipation layer 1303. The heat dissipation layer 1303 can have various structures, such as a circular shape, a rectangular shape or a flat plate shape. A cooling passage CP for the cooling medium to flow is formed in the heat dissipation layer 1303. The heat dissipation layer 1303 also has a cooling inlet IN (not shown) and a cooling outlet OUT (not shown). The cooling passage CP connects the cooling inlet IN and the cooling outlet OUT, and the cooling medium enters from the cooling inlet IN, passes through the cooling passage CP, and then exits from the cooling outlet OUT. The target T is irradiated by an accelerated proton beam with a high energy level to increase the temperature thereof and generate heat, and the heat dissipation layer conducts heat out, and the heat is taken out by the cooling medium flowing through the cooling passage, thereby cooling the target T.

In the embodiment, a first cooling tube D1 and a second cooling tube D2 are provided between the transmission tube C and the reflecting portion 1311 and the retarding portion 1312. One ends of the first cooling tube D1 and the second cooling tube D2 are respectively connected to the cooling inlet IN and the cooling outlet OUT of the target T, while the other ends thereof are connected to an external cooling source. The cooling medium can be deionized water, has an extremely low electrical conductivity, and prevents the generation of a leakage current in a high-voltage environment and an interference on the generation of the neutron beam. Alternatively, the first cooling tube and the second cooling tube can be disposed in the beam shaping portion in other manners, and when the target is arranged outside the beam shaping portion, the first cooling tube and the second cooling tube can also be cancelled. The target T can also include a supporting portion (not shown) for supporting or installing the target, and the supporting portion can also be configured for installing at least parts of the first cooling tube D1 and the second cooling tube D2. The cooling inlet IN and the cooling outlet OUT can also be arranged on the supporting portion. The supporting portion can be made of an aluminum alloy material, and a radioactive product of Al activated by neutrons has a short half-life, thereby reducing secondary radiation.

Although the service life of the target T is prolonged as much as possible by the above arrangements, the service life of the target T is still limited, and the target T needs to be replaced regularly or after loss. After a nuclear reaction occurs, the target, which a nuclear reaction has occurred on or has reaches its service life, is detached to be recycled and a new target is installed. The embodiments of the present application are intended to illustrate the installation of the new target, and the detachment and recycling of the old target will not be described in detail. In the embodiment, the material of the action layer 1301 is Li or alloys or compounds thereof, and due to the chemical property of Li metal is very active, so that it is easily oxidized or nitride, a target replacement chamber 40 can be provided to install a new target T, wherein the environment in the target replacement chamber 40 can be set as a dry environment, and Li will not be oxidized, nitrided or will be oxidized, nitrided slowly in a certain dry environment. In one embodiment, the dry environment has a dew point temperature of Td less than or equal to -30°C at atmospheric pressure or a relative humidity RH less than or equal to 0.6% at 25 °C.

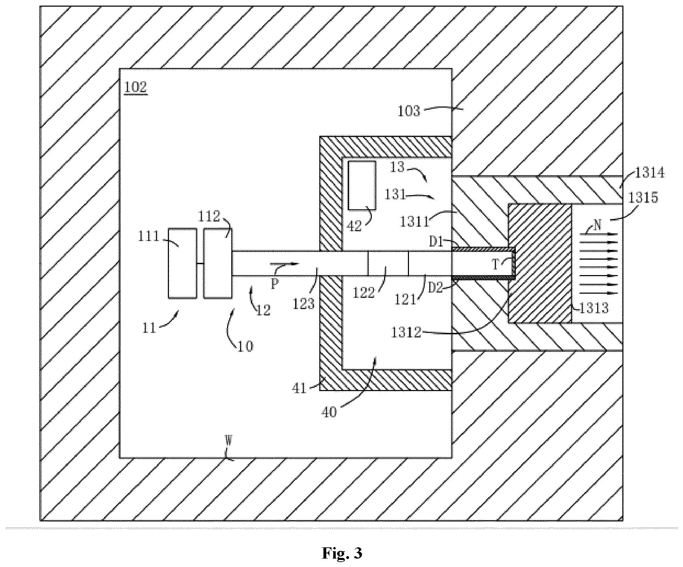

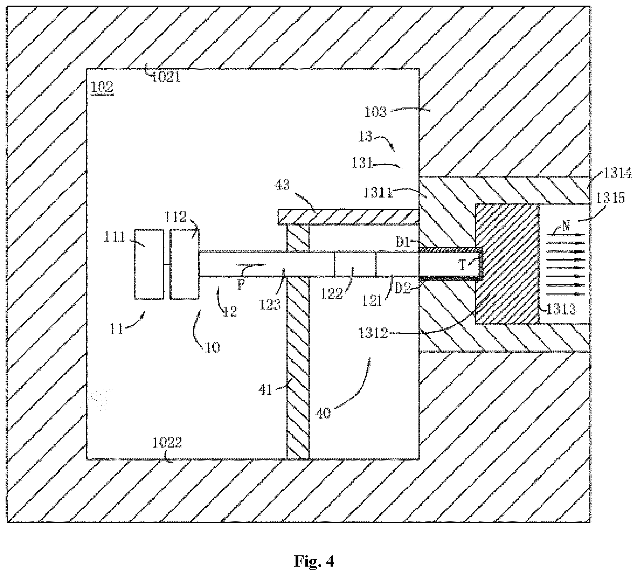

In one embodiment, the target replacement chamber 40 is configured for installing the target T and passing the beam transmission portion 12 through the target replacement chamber 40, and the target replacement chamber 40 is formed behind the charged particle beam generation portion 11 in the beam transmission path and is different from the charged particle beam generation chamber 102. Referring to Fig. 3, the target replacement chamber 40 is arranged in the charged particle beam generation chamber 102, and shares the separation wall 103 with the charged particle beam generation chamber 102. The charged particle beam generation chamber 102 further includes a ceiling plate 1021 and a floor plate 1022. In the embodiment, the target replacement chamber 40 is a semi-sealed dehumidification area, Here, the semi-sealed dehumidification area means that the formed space is not completely sealed, but after the dehumidification device is turned on, the corresponding space can form an environment meeting the requirement of use. The target replacement chamber 40 is enclosed by the separation wall 103, the ceiling plate 1021, the floor plate 1022 and the curtain 41 arranged between the ceiling plate 1021 and the floor plate 1022, and the dehumidifier 42 is arranged in this area. The curtain 41 is fixed on the ceiling plate 1021 and extends downwards to the floor plate 1022, and a cross section of the curtain 41 parallel to the ground can be in a shape of [-shaped, C-shaped, etc., the curtain 41 can include multiple curtain pieces arranged successively without gaps, and the curtain 41 has a low cost, is convenient to install, and is convenient to enter and leave, the provided dehumidifier 42 can also meet the requirement of dry environment. It can be understood that the material of the curtain 41 is PVC, the product generated by irradiation of the neutrons on the curtain 41 does not have radioactivity or has very low radioactivity, so that the generated secondary radiation is reduced. Alternatively, other materials which each, when irradiated by neutrons, generate a product having no radioactivity or low radioactivity, or a radioisotope with a short half-life can be used. The shell of the dehumidifier 42 can be made of a neutron shielding material or a radiation shielding member is provided outside the dehumidifier, so as to avoid radiation damage to the dehumidifier caused by neutrons generated during the neutron capture therapy. Alternatively, the target replacement chamber 40 can be enclosed only by the ceiling plate 1021, the floor plate 1022, and the curtain 41 arranged between the ceiling plate 1021 and the floor plate 1022, and in this case, the cross section of the curtain 41 parallel to the ground is a closed ring. The curtain 41 can also be enclosed by the ceiling plate 1021, the floor plate 1022, the curtain 41 arranged between the ceiling plate 1021 and the floor plate 1022 and a wall W adjacent to the separation wall 103, and in this case, the cross section of the curtain 41 parallel to the ground can be in a shape of linear-shaped or L-shaped. As shown in Fig. 4, the target replacement chamber 40 can further include a top 43 and a supporting portion (not shown) supporting the top 43, the supporting portion supports the top 43 on the ceiling plate 1021, the floor plate 1022, or a wall of the charged particle beam generation chamber 102. The curtain 41 is fixed to an outer edge of the top 43 and extends downwards to the floor plate 1022. The top 43, the floor plate 1022 and the curtain 41, or the top 43, the floor plate 1022, the curtain 41 and the separation wall 103 together enclose the target replacement chamber 40, and in this case, the target replacement chamber 40 can also be movable to a desired position only when a new target is installed. The target replacement chamber 40 can also be a drying room provided with a drying apparatus, a cleaning apparatus, a gas circulation system, etc., and can be arranged in the charged particle beam generation chamber 102, or the charged particle beam generation chamber 102 can provided as a drying room.

The transmission tube C can be adjustable in length to provide a space for replacement of the target T. The transmission tube C is a hollow tube in an internal vacuum state. The beam transmission portion 12 can include a first transmission tube 121, a second transmission tube 122, and a third transmission tube 123. The second transmission tube 122 is detachably connected to the first transmission tube 121 and the third transmission tube 123, respectively. The target T is arranged at the end of the first transmission tube 121 and can be detached together with the first transmission tube 121. Due to the target T has a large amount of radiation remained thereon after the nuclear reaction has carried out on the target T, people need to be prevented from touching as much as possible. In one embodiment, a control mechanism controls the first transmission tube 121 to be automatically removed into a recycling device (not shown) after the first transmission tube 121 is disconnected. The first transmission tube 121, the second transmission tube 122 and the third transmission tube 123 can remain in a vacuum state during the detaching process. In the installation state of the transmission tube C, the first transmission tube 121 and the second transmission tube 122 and a part of the third transmission tube 123 are arranged in the target replacement chamber 40, and the third transmission tube 123 passes through the target replacement chamber 40, for example, a hole on the curtain 41 corresponding to the third transmission tube 123. Alternatively, the first transmission tube 121 and a part of the second transmission tube 122 can be arranged in the target replacement chamber 40, the second transmission tube 122 passes through the target replacement chamber 40, and the third transmission tube 123 is provided outside the target replacement chamber 40. By disconnecting and removing the second transmission tube 122 from the first transmission tube 121 and the third transmission tube 123, the entire length of the transmission tube C is changed, thus leaving room for the first transmission tube 121 with the target T at the end thereof to move it out of the beam shaping portion 131. Alternatively, the length of the transmission tube C can be adjusted by other arrangements, such as the transmission tube C is a telescopic tube. A beam adjusting device can also be provided on the third transmission tube 123, instead of being provided on the first transmission tube or the second transmission tube, so as to avoid affecting beam or damaging the beam adjusting device during replacing the target T.

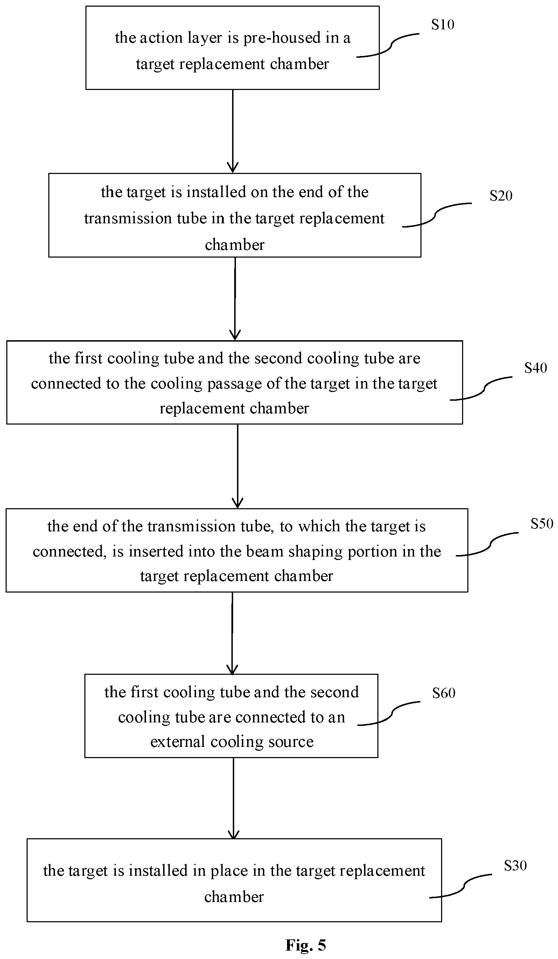

After the used target T is recycled, a new target is installed in the target replacement chamber 40. As shown in Fig. 5, a method for installing the target T may include the following steps 10 to 30:

In step 10, the action layer 1301 is pre-housed in the target replacement chamber 40, for example, before installing, the sealed target T provided with the action layer 1301 is taken out from a storage chamber (not shown), placed the target T in the target replacement chamber 40, and opened in the target replacement chamber 40;

In step 20, the target T is installed on the end of the transmission tube C in the target replacement chamber 40, for example, the target T is installed on the end of the first transmission tube 121 via the supporting portion; and

In step 30, the target T is installed in place in the target replacement chamber 40, for example, the first transmission tube 121 is connected to the remaining parts of the transmission tube C and the length of the remaining parts of the transmission tube C is adjusted. In the embodiment, the detached second transmission tube 122 is connected between the first transmission tube 121 and the third transmission tube 123.

In the embodiment, the target T is inserted into the beam shaping portion 131, and the first cooling tube D1 and the second cooling tube D2 are provided between the transmission tube C and the beam shaping portion 131 and connected to the cooling passage CP of the heat dissipation layer 1303 of the target T. The following steps 40 to 60 further are between step 20 and step 30:

In step 40, the first cooling tube D1and the second cooling tube D2 are connected to the cooling passage CP of the target T in the target replacement chamber 40;

In step 50, the end of the transmission tube C connected with the target T is inserted into the beam shaping portion 131 in the target replacement chamber 40; and

In step 60, the first cooling tube D1and the second cooling tube D2 are connected to an external cooling source;

Alternatively, step 60 can be performed after step 30 or step 40, step 60 can be performed in or outside the target replacement chamber 40, and the first cooling tube D1 and the second cooling tube D2 can be fixed on the first transmission tube 121. The first transmission tube 121 and the target T can also be integrally packaged, so as to improve the target replacement efficiency. The whole of the target T and the first transmission tube 121 is pre-housed in the target replacement chamber 40, and is opened and connected to the first cooling tube D1 and the second cooling tube D2 in the target replacement chamber 40. The first cooling tube D1 and the second cooling tube D2 can also be at least partially integrally packaged with the first transmission tube 121, and a detection device, such as a target temperature detection device, a neutron or γ radiation detection device, can also be provided on the first transmission tube 121.

It can be understood that the present disclosure can also be applied to neutron beam generation devices or other particle beam generation devices in which the action layer of the target is other materials easily oxidized and nitrided, and can also be applied to other medical and non-medical fields.

Although the illustrative embodiments of the present disclosure have been described above to facilitate understanding of the present disclosure by those skilled in the art, however, it should be clear that the present disclosure is not limited to the specific embodiments, as long as such variations are within the spirit and scope of the disclosure as defined and determined by the appended claims, these variations are obvious and are within the scope of the present disclosure.

## Claims

1. A neutron capture therapy system, comprising a charged particle beam generation portion, a beam transmitting portion, and a neutron beam generation portion, wherein the charged particle beam generation portion is configured for generating a charged particle beam, the beam transmission portion transmits the charged particle beam to the neutron beam generation portion, the neutron beam generation portion comprises a target that interacts with the charged particle beam to generate a neutron beam,
the neutron capture therapy system further comprises a charged particle beam generation chamber and a target replacement chamber, wherein the charged particle beam generation chamber at least partly houses the charged particle beam generation portion and the beam transmission portion, the target replacement chamber is configured for installing the target and passing the beam transmission portion through the target replacement chamber, and the target replacement chamber is formed behind the charged particle beam generation portion in a transmission path of the charged particle beam and is different from the charged particle beam generation chamber.

2. The neutron capture therapy system according to claim 1, wherein the target comprises an action layer, the action layer interacts with the charged particle beam to generate the neutron beam, and the action layer is pre-housed in the target replacement chamber.

3. The neutron capture therapy system according to claim 2, wherein a material of the action layer is Li or an alloy thereof or a compound thereof, the charged particle beam is a proton beam, and the proton beam is performed a nuclear reaction of ⁷Li(p, n)⁷Be with the action layer to generate the neutron beam.

4. The neutron capture therapy system according to claim 1, wherein an environment in the target replacement chamber has a dew point temperature of Td less than or equal to -30°C at atmospheric pressure or a relative humidity RH less than or equal to 0.6% at 25 °C.

5. The neutron capture therapy system according to claim 1, wherein the target comprises a cooling passage, and the neutron capture therapy system further comprises a first cooling tube and a second cooling tube both connected to the cooling passage.

6. The neutron capture therapy system according to claim 1, wherein the neutron beam generation portion further comprises a beam shaping portion capable of adjusting a quality of the neutron beam generated by an interaction of the charged particle beam and the target; the neutron capture therapy system further comprises an irradiation chamber in which a subject to be irradiated is performed therapy by irradiation of the neutron beam, the beam shaping portion is at least partially housed in a separation wall configured for separating the irradiation chamber and the charged particle beam generation chamber, the target is installed in the beam shaping portion, and the target replacement chamber is arranged in the charged particle beam generation chamber.

7. The neutron capture therapy system according to claim 6, wherein the charged particle beam generation chamber has a ceiling plate and a floor plate, the target replacement chamber is a semi-sealed dehumidification area enclosed by the separation wall, the ceiling plate, the floor plate, and a curtain arranged between the ceiling plate and the floor plate, and a dehumidifier is arranged in the semi-sealed dehumidification area; alternatively, the target replacement chamber comprises a top, the target replacement chamber is a semi-sealed dehumidification area enclosed by the separation wall, the top, the floor plate and a curtain arranged between the top and the floor plate, and a dehumidifier is arranged in the semi-sealed dehumidification area.

8. The neutron capture therapy system according to claim 7, wherein the curtain comprises a plurality of curtain pieces sequentially arranged without gaps; the curtain is made of a material which, when irradiated by neutrons, generates a product having no radioactivity or low radioactivity, or a radioisotope with a short half-life; the dehumidifier comprises a shell which is made of a neutron shielding material or is provided with a radiation shield outside the dehumidifier.

9. The neutron capture therapy system according to claim 1, wherein the beam transmission portion comprises a transmission tube having an adjustable length, the target is arranged on an end of the transmission tube, and the end of the transmission tube on which the target is arranged is located in the target replacement chamber.

10. The neutron capture therapy system according to claim 9, wherein the beam transmission portion comprises a first transmission tube, a second transmission tube and a third transmission tube, wherein the second transmission tube is detachably connected to the first transmission tube and the third transmission tube, and the target is provided on an end of the first transmission tube, the first transmission tube and at least part of the second transmission tube are arranged in the target replacement chamber, the second transmission tube or the third transmission tube passes through the target replacement chamber.

11. The neutron capture therapy system according to claim 10, wherein a beam adjusting device is disposed on the third transmission tube, and a detection device is disposed on the first transmission tube.

12. An installing method for a target of a particle beam generation device, the target comprises an action layer configured for generating a particle beam, the particle beam generation device comprises a transmission tube, the target is installed on an end of the transmission tube, the installing method comprises:
pre-housing the action layer in a target replacement chamber;
installing the target on the end of the transmission tube in the target replacement chamber;
installing the target to the particle beam generation device in the target replacement chamber.

13. The installing method according to claim 12, wherein the transmission tube comprises a first transmission tube and a second transmission tube, the target is installed on an end of the first transmission tube, and a length of the second transmission tube is adjustable, the target is installed to the particle beam generation device by connecting the first transmission tube to the second transmission tube and adjusting the length of the second transmission tube in the target replacement chamber.

14. The installing method according to claim 12, wherein the particle beam generation device further comprises a beam shaping portion, the target is arranged in the beam shaping portion, the target comprises a cooling passage, the particle beam generation device further comprises a first cooling tube and a second cooling tube both connected to the cooling passage, the installing method further comprises:
connecting the first cooling tube and the second cooling tube to the cooling passage of the target in the target replacement chamber;
inserting the end of the transmission tube, on which the target is installed, into the beam shaping portion in the target replacement chamber;
connecting the first cooling tube and the second cooling tube to an external cooling source.

15. The installing method of claim 12, wherein an environment in the target replacement chamber has a dew point temperature of Td less than or equal to -30°C at atmospheric pressure or a relative humidity RH less than or equal to 0.6% at 25 °C.
